# EUROPEAN PATENT SPECIFICATION

(11) **EP 1 141 153 B1**
(45) Date of publication and mention of the grant of the patent: **10.09.2003**
(21) Application number: 99972240.8
(22) Date of filing: 06.11.1999
(51) Int. Cl.: C09D 201/00, C08K 5/3492, C07D 405/14

(54) **CROSSLINKING COMPOSITION FOR CARBOXYL-CONTAINING POLYMERS IN HEAT-CURABLE SYSTEMS**
ZUSAMMENSETZUNG ZUM VERNETZEN VON CARBOXYL-HALTIGEN POLYMEREN IN THERMISCH HÄRTBAREN SYSTEMEN
COMPOSITION POUR LA RETICULATION DE POLYMERES CONTENANT DU CARBOXYLE DANS DES SYSTEMES THERMODURCISSABLES

(30) Priority: 17.11.1998 CH 229998
(43) Date of publication of application: 10.10.2001
(73) Proprietor: Vantico AG, 4057 Basel (CH)
(72) Inventor: RICKERT, Christoph, CH-4153 Reinach (CH); FRANCOIS, Jacques, F-68300 Saint Louis (FR); PICOT, Jean-Bernard, F-92160 Antony (FR)
(74) Representative: Dannappel, Hans-Jochen, Dr.
(86) International application number: EP9908530
(87) International publication number: WO00029497

(56) References cited:
- EP-A- 0 529 361
- WO-A-97/24408
- DE-A- 4 129 752

## Description

The present invention relates to crosslinking compositions (hardeners) for heat-curable systems containing carboxyl-containing polymers, in particular to systems containing carboxyl-terminated polyester polymers, carboxyl-containing acrylate polymers and/or carboxyl-containing methacrylate polymers. This invention relates in particular to a crosslinking agent based on cyclocarbonate group-containing isocyanurate compounds.

It is known to use cyclocarbonate groups containing isocyanurates to replace the analogous polyglycidyl compounds as crosslinking agents in curable compositions containing carboxyl-containing polymers, such as carboxyl-terminated polyesters, carboxyl-containing acrylates and/or methacrylate polymers. WO 97/24408, for example, discloses curable systems containing at least one carboxyl-terminated polyester compound as binder, one cyclocarbonate group-containing isocyanurate compound and one polyglycidyl compound as crosslinking agent, an accelerator (catalyst) as well as other per se known additives. The use of a cyclocarbonate group-containing isocyanurate compound is regarded as an alternative for using corresponding polyglycidyl compounds. As a consequence of the curing process, carbon dioxide (CO₂) is liberated from the cyclocarbonate group and escapes, which may result in foam formation at coating thicknesses of more than 20 µm, unless the composition contains a certain amount of at least one glycidyl compound. The presence of the glycidyl compound results in a reduction of the foam formation so that coating thicknesses of up to 100 µm can be produced without any substantial interfering foam formation. However, there still remain the different reaction rates of the two crosslinking agents towards the binder so that it is necessary to carry out curing at relatively high temperatures. In consequence, the curing of the glycidyl groups proceeds relatively quickly, which impedes the carbon dioxide's escape without bubble formation. If the cyclocarbonate group-containing compounds have a melting point above 120°C or 130°C and if these compounds are furthermore sparingly soluble or insoluble in the binder, then the mentioned disadvantages are augmented by the different reaction rates of the two crosslinking agents with the binder.

Surprisingly, it has now been found that the cited disadvantages are substantially or completely overcome if the catalyst to be used for crosslinking the cyclocarbonate groups is incorporated in the cyclocarbonate group-containing isocyanurate compound beforehand by a suitable method and if the crosslinking agent thus modified is only then added to the curable system. In this manner it is possible to start curing the system at a comparatively low temperature, often below the melting point of the cyclocarbonate group-containing compounds. Moreover, less catalyst is required, which reduces the costs. The carbon dioxide can escape at a lower temperature, i.e. when curing is less advanced, substantially reducing the foaming tendency. The escape of the carbon dioxide can start at a lower temperature and stretch over a longer period of time, which in turn makes it possible to produce thicker coatings, i.e. coating thicknesses of more than 100 µm and, where necessary, of up to 200 µm, without foam formation.

The present invention is defined in the patent claims. This invention relates in particular to a crosslinking composition for heat-curable carboxyl-containing polymers, in particular for systems containing carboxyl-terminated polyesters, carboxyl-containing acrylate polymers and/or methacrylate polymers, comprising a crosslinking agent, which consists at least of one cyclocarbonate group-containing isocyanurate compound, and at least one catalyst dissolved or dispersed therein, which has been incorporated separately into the isocyanurate compound prior to the crosslinking reaction.

The crosslinking composition of the present invention is preferably prepared by dissolving a cyclocarbonate group-containing isocyanurate compound in a suitable solvent, dissolving or dispersing therein at least one catalyst and then removing the solvent again.

Accordingly, this invention relates to a process for the preparation of the crosslinking composition of the present invention, which comprises dissolving a cyclocarbonate group-containing isocyanurate compound in a suitable solvent, dissolving or dispersing therein at least one catalyst and then removing the solvent again. This invention also relates to the crosslinking agents which are prepared or obtainable in this manner.

This invention also relates to the use of the crosslinking composition of the present invention as hardener in heat-curable carboxyl-containing polymers, in particular in systems containing carboxyl-terminated polyesters, carboxyl-containing acrylate and/or methacrylate polymers, preferably in heat-curable paint systems and, especially, in heat-curable powder coating compositions.

Such heat-curable systems, paint systems or powder coating compositions preferably comprise (i) as binder at least one carboxyl-containing polymer, more preferably a carboxyl-terminated polyester and/or a carboxyl-containing acrylate and/or methacrylate polymer, (ii) a novel crosslinking agent, (iii) optionally one or several glycidyl compound(s) and at least one catalyst for the crosslinking reaction of said glycidyl compound(s) together with the carboxyl-containing polymer, and (iv) per se customary other additives.

The cyclocarbonate groups contained in the crosslinking agent can be prepared by reacting the corresponding glycidyl group-containing isocyanurate compound with carbon dioxide in the presence of a catalyst, preferably a basic compound, part or all of the glycidyl groups present in the molecule converting into the cyclocarbonate group. During the crosslinking reaction with the carboxyl group, i.e. during the curing process, the cyclocarbonate group releases the carbon dioxide absorbed during the synthesis. The preparation of the cyclocarbonate group from the glycidyl group is known per se and is described, inter alia, in WO 97/24408, pages 6 and 7 with additional source references, as cited at the outset.

The cyclocarbonate group-containing isocyanurate compounds which may be used according to this invention preferably have a melting point of at least 120°C, more preferably of at least 130°C and, particularly preferably, of at least 140°C, and are normally sparingly soluble or insoluble in the binder, i.e. their solubility is below 20 gram, and often below 10 gram, of isocyanurate compound per 100 gram of binder.
The cyclocarbonate group-containing isocyanurate compound is preferably a tris(2-oxo-1,3-dioxolanyl-4-methyl)isocyanurate according to the following formula (I):

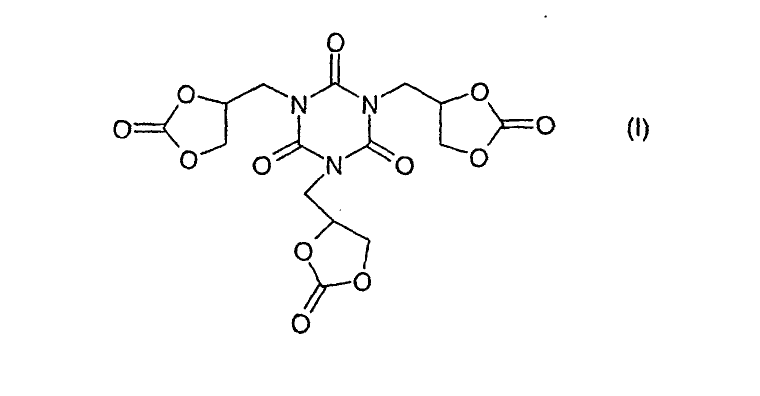

The compound of formula (I) is listed in the form which is usually preferred. In analogy to the compound of formula (I) it is also possible to use a compound which contains only one single or two cyclocarbonate group(s), the remaining groups being in the form of glycidyl radicals. The compound of formula (I) preferably contains not more than 35 mol-% of epoxy groups, particularly preferably not more than 5 mol-%.

The crosslinking composition of the present invention which contains at least one catalyst is preferably prepared by dissolving a cyclocarbonate group-containing isocyanurate compound in a suitable solvent, dissolving or dispersing therein at least one catalyst and then removing the solvent again. The solvents used may be suitable inorganic and organic solvents, for example lactones, dimethylsulfoxide, amidic solvents, such as N-methylpyrrolidone or dimethylformamide. A preferred solvent is γ-butyrolactone.

To achieve a sufficient crosslinking reaction, the crosslinking composition of the present invention contains the catalyst in an amount from 0.01 % by weight to 20 % by weight, preferably from 0.1 % by weight to 10 % by weight and, more preferably, from 5 % by weight to 10 % by weight, based on the weight of the cyclocarbonate group-containing isocyanurate compound.

The crosslinking composition of the present invention, for example the tris(2-oxo-1,3-dioxolanyl-4-methyl)isocyanurate compound, into which e.g. an ethyltriphenylphosphonium bromide has been incorporated in a concentration of 0.01 to 20 % by weight (based on the weight of the cyclocarbonate group-containing isocyanurate compound), is used in concentrations from 1 to 20 % by weight, preferably in concentrations from 2 to 10 % by weight, based on the sum of the weight of crosslinking agent and binder.

Besides this crosslinking composition, the curable system preferably also contains at least one crosslinking glycidyl compound. The weight ratio of the amount of the crosslinking composition of the present invention on the one hand (calculated on the weight of the cyclocarbonate group-containing isocyanurate compound(s) present therein) to the amount of the crosslinking glycidyl compound(s) on the other hand, is preferably from 0.1 to 2.0, more preferably from 0.1 to 1.0. A ratio from 0.2 to 0.5 is particularly preferred. The crosslinking glycidyl compound is in this case added to the curable composition separately, and the corresponding catalyst is also added to the composition separately. The kind and amount of the separately added catalyst is adjusted to, or calculated for, the kind and amount of the separately added glycidyl compound. The catalysts used for the glycidyl compounds are preferably added in per se known concentrations used for conventional heat-curable systems based on carboxyl-terminated polyesters and glycidyl compounds.

According to this invention it is thus preferred to use on the one hand a catalyst which acts selectively predominantly or exclusively on the cyclocarbonate group-containing isocyanurate compound, the respective catalyst being separately incorporated to this purpose into the isocyanurate compound prior to the crosslinking reaction. On the other hand, a catalyst is also added to the curable composition which acts predominantly and preferably selectively on the glycidyl compound.

Examples of suitable accelerators (catalysts) which accelerate the crosslinking reaction of the cyclocarbonate group-containing isocyanurate compounds with the carboxyl-containing polymers and the liberation of carbon dioxide during the corresponding crosslinking, are in particular compounds acting as Lewis acids and Lewis bases and also certain inorganic salts and their hydrates. Corresponding examples are FeSO₄, NaHSO₄, CeSO_{4,} H₃PO₄, ZnCl₂, Na₂CO₃, phosphonic acid, p-toluenesulfonic acid and dimethylsulfonic acid. Ammonium salts and/or phosphonium salts are preferred. Examples are quaternary ammonium salts such as tetraalkyl ammonium halides, aryl- and alkyl-substituted ammonium halides, such as tetramethyl ammonium bromide, trimethylbenzyl ammonium hydroxide, 2-hydroxypyridine, trimethylbenzyl ammonium methoxide, phenyltrimethyl ammonium chloride, phenyltrimethyl ammonium bromide, phenyltrimethyl ammonium hydroxide, phenyltrimethyl ammonium iodide, phenyltrimethyl ammonium tribromide, the sodium salt of phosphocholine chloride, stearyl ammonium bromide, tetra-n-amyl ammonium iodide, tetra-n-butyl ammonium bromide, tetra-n-butyl ammonium hydroxide, tetra-n-butyl ammonium phosphate, tetra-n-decyl ammonium trichloride, tetraethyl ammonium hydroxide, tetraethyl ammonium tetrafluoroborate, tetramethylguanidine, acetylcholine bromide, alkyldimethylbenzyl ammonium chloride, benzylcholine bromide, benzyl-n-butyl ammonium bromide, bis(tetra-n-butyl ammonium)dichromate, trimethyl vinyl ammonium bromide; phosphonium salts, for example ethyltriphenylphosphonium bromide, DBU (1,8-diazabicyclo(5.4.0)undec-7-ene(1,5-5), tetramethyl ammonium chloride (TMAC), allyltriphenylphosphonium chloride, benzyltriphenylphosphonium chloride, bromomethyltriphenylphosphonium bromide, 2-dimethylaminoethyltriphenylphosphonium bromide, ethoxycarbonylphosphonium bromide, n-heptyltriphenylphosphonium bromide, methyltriphenylphosphonium bromide, tetrakis(hydroxymethyl)phosphonium sulfate and tetraphenylphosphonium bromide. Phosphonium salts are preferred, in particular the cited phosphonium halides. Ethyltriphenylphosphonium bromide is particularly preferred.

As mentioned above, the curable system preferably contains besides the novel crosslinking agent also at least one crosslinking glycidyl compound. Preferred crosslinking glycidyl compounds are those which contain at least two 1,2-epoxy groups in the molecule and which may also be called polyglycidyl compound(s). It is preferred to use a mixture of polyglycidyl compounds, for example a mixture of diglycidyl and triglycidyl compounds. Such compounds are known per se and are described in the literature. A suitable selection can usually be made from the known glycidyl compounds.

In this invention it is preferred to use glycidyl compounds such as those described, inter alia, in EP-A-0 297 030, EP-A-0 356 391, EP-A-0 462 053, EP-A-0 506 617 and EP-A-0 536 085. These include compounds containing unsubstituted glycidyl groups and/or methyl group-substituted glycidyl groups. The glycidyl compounds preferably have a molecular weight from 200 to 1200, more preferably from 200 to 1000, and may be solid or liquid. Their epoxy content is preferably at least three equivalents per kilogram of the compound, preferably at least four equivalents per kilogram and, more preferably, at least five equivalents per kilogram. Preferred glycidyl compounds are those which contain glycidyl ether and/or glycidyl ester groups. A glycidyl compound may in this case also contain both kinds of glycidyl groups, for example 4-glycidyloxybenzoic acid glycidyl ester. Polyglycidyl esters containing 1-4 glycidyl ester groups are preferred, in particular diglycidyl ester and/or triglycidyl ester.

The preferred diglycidyl esters are preferably derived from aromatic, araliphatic, cycloaliphatic, heterocyclic, heterocyclic-aliphatic or heterocyclic-aromatic dicarboxylic acids containing 6 to 20, preferably 6 to 12, ring carbon atoms, or from aliphatic dicarboxylic acids containing 2 to 10 carbon atoms. Compounds of this type are commonly known and are described, inter alia, in U.S. patent US-A-3,859,314 or in DE-A-31 26 411. Examples of suitable dicarboxylic acids are phthalic acid, isophthalic acid, terephthalic acid, 2,5-dimethylphthalic acid, 5-tert-butylisophthalic acid, naphtalene-2,6-dicarboxylic acid, naphtalene-1,8-dicarboxylic acid, naphthalene-2,3-dicarboxylic acid, diphenyl ether-4,4'-dicarboxylic acid, diphenyl-2,2'-dicarboxylic acid, tetrachlorophthalic acid, 2,5-dichlorophthalic acid, ortho-, metha- or para-phenylenediacetic acid, oxalic acid, malonic acid, succinic acid, adipic acid, 2,2,4-trimethyladipic acid, 2,4,4-trimethyladipic acid, sebacic acid, azelaic acid, fumaric acid, maleic acid and compounds such as the dicarboxylic acids obtainable by adding acrylonitrile or acrylate to compounds containing hydrogen atoms which can be activated, such as ketones, nitrogen compounds, diols or dithiols; tetrahydrophthalic acid, methyltetrahydrophthalic acid, hexahydrophthalic acid, methylhexahydrophthalic acid, endomethylenehexahydrophthalic acid, hexahydroterephthalic acid, hexahydroisophthalic acid, thiophene-2,5-dicarboxylic acid, furan-2,5-dicarboxylic acid, furan-3,4-dicarboxylic acid, pyrazine-3,4-dicarboxylic acid; 1,3-bis(carboxyethyl)hydantoin, 1,1-methylenebis[3-(p-glycidyloxycarbonylbenzyl)-5,5-dimethylhydantoin], each of which is unsubstituted or alkyl-substituted in 5-position, and other dicarboxylates containing one or several hydantoin ring(s), and N,N'-bis(p-glycidyloxycarbonylbenzoyl)isophoronediamine.

Particularly preferred are terephthalic acid diglycidyl ester or isophthalic acid diglycidyl ester or 1,4-hexahydrophthalic acid diglycidyl ester or oxalic acid diglycidyl ester or adipic acid diglycidyl ester or sebacic acid diglycidyl ester or azelaic acid diglycidyl ester or succinic acid diglycidyl ester.

Preferred are also glycidyl esters containing at least three glycidyl groups per molecule, for example polyglycidyl ester containing three or four glycidyl groups, in particular trimellitic acid triglycidyl ester, trimesic acid triglycidyl ester and pyromellitic acid tetraglycidyl ester. The preparation of the cited glycidyl compounds is known per se. Preferred glycidyl compounds and their combinations are described, inter alia, in P.-G. Gottis, J.-A. Cotting, FATIPEC Congress (1996), 23^{rd} (Vol.B), B216-B231 (ISSN:0430-222), "Solid solutions of glycidyl compounds as TGIC alternatives in polyester powder coatings".

It is particularly preferred to use combinations of tris(2-oxo-1,3-dioxolanyl-4-methyl)isocyanurate with a mixture of a diglycidyl compound and a triglycidyl compound, for example a mixture of diglycidyl terephthalate and trimellitic acid triglycidyl ester, and to use these compounds in a weight ratio of diglycidyl compound to triglycidyl ester of 4 : 1 to 1 : 10, and preferably of about 3 : 1.

In principle, the above catalysts for the reaction of the cyclic carbonates with carboxylic acids can also be used for the glycidyl compounds. Examples of preferred catalysts which accelerate the crosslinking reaction of the glycidyl compounds with the carboxyl-containing polymers are, however, Actiron® NXJ-60 (2-propylimidazole), Actiron® NXJ-60 P (60 % by weight 2-propylimidazole on 40 % by weight of solid substrate), Accelerator® DT 3126 (a commercially available and per se known alkyl ammonium bromide masterbatch), triphenylphosphine or ethyltriphenylphosphonium bromide, in order to enable a sufficiently fast curing reaction even at relatively low temperatures, e.g. in the range from 60°C to 160°C. These catalysts are often an organic amine or a derivative of an amine, preferably a tertiary amine or an ammonium salt or a nitrogen-containing heterocyclic compound. Preferred catalysts for the reaction of epoxy groups with carboxyl groups are phenylimidazole, N-benzyldimethylamine and 1,8-diazabicyclo[5,4,0]-7-undecene, where required on a substrate, e.g. silicate substrate. The catalyst, or a catalyst mixture, is usefully added in an amount of about 0.1 to 10 % by weight, preferably of 0.5 to 5 % by weight and, most preferably, of about 0.4 % by weight, based on the weight of the glycidyl compound.

The above-described components are used in heat-curable compositions, in particular in paint systems, preferably in powder coating compositions, which contain as binder a carboxyl-containing polymer, preferably a carboxyl-terminated polyester and/or a carboxyl-containing acryl resin, reacting with the crosslinking composition(s) with crosslinking.

The acryl resin is in this case an acrylate polymer or a methacrylate polymer, preferably a copolymer of one or more than one acrylate and/or methacrylate, preferably of the corresponding alkyl esters containing 1 to 18, preferably 1 to 8, carbon atoms in the alkyl group, with acrylic acid and/or methacrylic acid and, optionally, with additional ethylenically unsaturated comonomers, and has e.g. a molecular weight (average number Mn from GPC measurement with polystyrene calibration) of 500 to 30000, preferably of 1000 to 10000. Said acryl resin furthermore preferably contains 0.2 to 6 equivalents of free carboxyl groups. The glass transition temperature of the acrylate polymers and methacrylate polymers is usefully above 20°C and is preferably in the range from 30 to 100°C. Examples of suitable (meth)acrylate monomers are ethyl acrylate, butyl acrylate, 2-ethylhexyl acrylate and, in particular, C₁-C₄alkylmethacrylates, such as methyl methacrylate, ethyl methacrylate or butyl methacrylate. (Meth)acrylate derivatives containing the silane groups can also be used. Suitable ethylenically unsaturated comonomers are, for example, acrylo- or methacrylonitriles and vinyl compounds. Preferred comonomers are vinyl aromatic compounds, in particular styrenes. The above-cited polymers can be prepared in known manner, for example by polymerising the monomers dissolved in suitable organic solvents, preferably in toluene or in mixtures of 1-methoxy-2-propanol, 1-methoxy-2-propyl acetate and methyl isobutyl ketone (for example at a weight ratio of 70/20/10) in the presence of a suitable initiator, for example dicumyl peroxide, and of a chain transfer reagent, such as thioglycolic acid. They can also be polymerised in the melt. The carboxyl group-containing polyester polymers preferably have an acid number (indicated in mg KOH/g polyester) of 10 to 100 and a molecular weight (number average Mn) of 2000 to 10000. The ratio Mw (weight average of the molecular weight) to Mn in these polyesters is usually from 2 to 10. The polyesters are usefully solid at room temperature and preferably have a glass transition temperature from 35 to 120°C, more preferably from 40 to 80°C. They are condensates of polyols with dicarboxylic acids and, optionally, of polyfunctional carboxylic acids or the corresponding carboxylic anhydrides. Suitable polyols are, for example, ethylene glycol, diethylene glycol, the propylene glycols, butylene glycols, 1,3-butanediol, 1,4-butanediol, neopentyl glycol, isopentyl glycol, 1,6 hexanediol, glycerol, hexanetriol, trimethylolethane, trimethylolpropane, erythritol, pentaerythritol, cyclohexanediol or 1,4-dimethylolcyclohexane. Suitable dicarboxylic acids are, for example, isophthalic acid, terephthalic acid, phthalic acid, methylphthalic acids, tetrahydrophthalic acid, hexahydrophthalic acid methyltetrahydrophthalic acids, e.g. 4-methyltetrahydrophthalic acid, cyclohexanedicarboxylic acids, succinic acid, glutaric acid, adipic acid, pimelic acid, suberic acid, azelaic acid, sebacic acid, dodecanedicarboxylic acid, fumaric acid, maleic acid or 4,4'-diphenyldicarboxylic acid, and the like. Suitable tricarboxylic acids are, for example, aliphatic tricarboxylic acids, such as 1,2,3-propanetricarboxylic acid, aromatic tricarboxylic acids, such as trimesic acid, trimellitic acid and hemimellitic acid, or cycloaliphatic tricarboxylic acids, such as 6-methylcyclohex-4-ene-1,2,3-tricarboxylic acid. Suitable tetracarboxylic acids are, for example, pyromellitic acid or benzophenone-3,3',4,4'-tetracarboxylic acid. Commercially available polyesters are often based on neopentyl glycol and/or trimethylolpropane as essential alcoholic components and on adipic acid and/or terephthalic acid and/or isophthalic acid and/or trimellitic acid as essential acid components.

The curable compositions of the present invention, can furthermore contain other customary additives, for example light stabilisers, colourants, pigments, e.g. titanium dioxide, degassing agents, e.g. benzoin, adhesives, thixotropic agents and/or flow control agents. The novel curable compositions may also contain a suitable inert solvent or solvent mixture, for example xylene, butyl acetate, isobutanol, 1-methoxy-2-propanol, 1-methoxy-2-propylacetate or methyl isobutyl ketone (MIBK).

The novel curable compositions may be used in the conventional technological fields of curable epoxy resin compositions, in particular for paint systems, preferably for powder coating compositions. Powder coating compositions can be prepared by simple blending of the components, e.g. in a ball mill. Another and more preferred possibility is to melt the components together and to blend them until they are homogeneous, preferably in an extruder, e.g. in a Buss Co-kneader, and then to cool the melt and comminute it. The powder coating composition mixtures preferably have an average particle size in the range from 0.015 to 500 µm, preferably from 5 to 100 µm.
Depending on the application, the powder coating compositions are cured on the article to be coated at a temperature of at least 100°C, preferably in the range from 150°C to 250°C. Curing usually requires about 5 to 60 minutes. Materials suitable for coating are all materials which are stable at the temperatures required for curing, in particular metals and ceramics.
In particular where polyesters building units are used which consist to 50 % by weight, preferably to 90 % by weight and more, of neopentanediol and aromatic or cycloaliphatic dicarboxylic acids, preferably of terephthalic acid, and which are commercially available e.g. as Crylcoat® types (UCB) or under names such as Uralac® (DSM), Alftalat® (Vianova) or Grilesta® (EMS), powder coating compositions are obtained which yield weather-resistant and particularly flexible coatings which are suitable for outdoor applications, which is the case both under sudden and under prolonged mechanical stress.

The following Examples illustrate the invention.

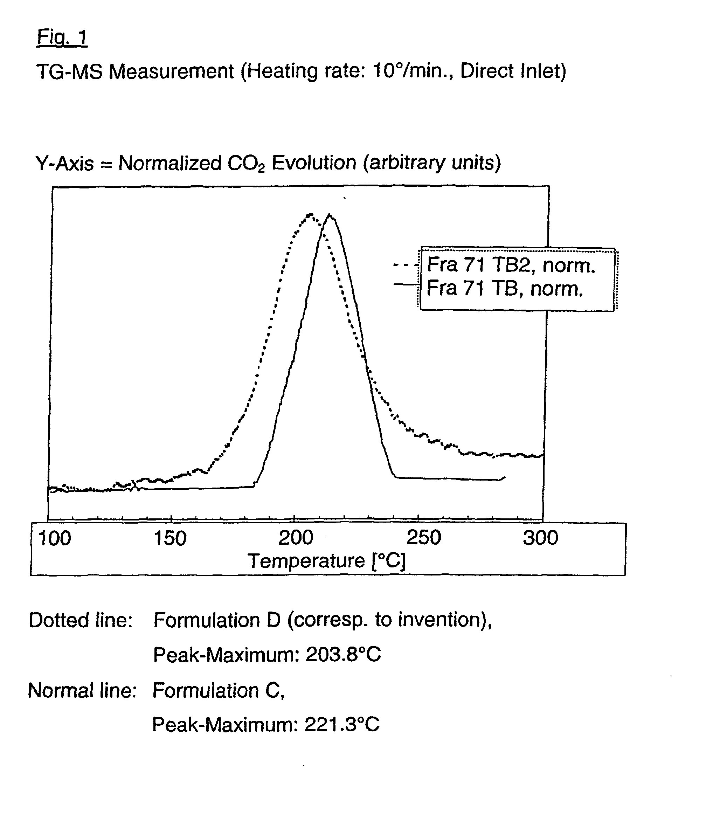

Figure 1 shows a comparison between the carbon dioxide liberation when (i) using tris(2-oxo-1,3-dioxolanyl-4-methyl)isocyanurate and ethyltriphenylphosphonium bromide separately in accordance with formulation C, and (ii) when using the novel formulation D.

### Example 1 (Preparation of a crosslinking agent (tris[2-oxo-1,3-dioxolanyl-4-methyl]isocyanurate) which comprises the catalyst (ethyltriphenylphosphonium bromide, ETPPBr) in dissolved form.)

20 Parts per weight of tris(2-oxo-1,3-dioxolanyl-4-methyl)isocyanurate (TGIC-CO₂), prepared according to WO 97/24408, are dissolved, with stirring, in 200 parts per weight of γ-butyrolactone (Fluka 20741) at 130°C. Subsequently, 1 part per weight of ethyltriphenylphosphonium bromide (Chemconserve CV, P.O.B. 566, 2280 AN Rijswijk, Netherlands), corresponding to a catalyst concentration of 5 % by weight, are added and the mixture is stirred at the same temperature until it is completely dissolved. The solution so obtained is concentrated to dryness in a rotary evaporator, yielding the product, i.e. a novel crosslinking agent, in the form of a fine powder.

### Example 2 (Use of the product of Example 1 as crosslinking agent for powder coating compositions based on polyesters.)

a) The substances listed in Table 1 under formulation F are mixed in the cited amounts in a mixer, using, as stated in Table 1, 2.33 % by weight of the novel crosslinking agent prepared in Example 1. To homogenise the mixture, it is extruded twice at 90°C in a twinscrew extruder (Prism TSE 16 PC). The cooled extrudate is ground in an ultracentrifugal mill (Retsch ZSM 1000) to an average particle size of about 40 µm. Particles larger than 100 µm are removed by sieving. The gel time of the powder coating composition at 180°C (in accordance with ISO norm 8130) is 240 seconds. The powder coating composition is sprayed electrostatically on test panels (Q-panel, of Q-Panel). The corresponding stoving conditions and coating thicknesses and the resulting properties of the coatings are compiled in Table 2, under F. Additional properties are listed in Table 3.
b) The powder coating formulations B, D, E and G listed in Table 1 are prepared in analogy to Example 1a).The stoving conditions, coating thicknesses and properties of the coatings are also listed in Table 2, under B, D, E, and G, respectively. Additional properties are compiled in Table 3.

### Example 3 (Comparison Example)

The process described in Example 2 is repeated (i) using the formulation C and (ii) using the formulation A, each according to Table 1, tris(2-oxo-1,3-dioxolanyl-4-methyl)isocyanurate and ethyltriphenylphosphonium bromide being added separately to the formulation. The corresponding stoving conditions, coating thicknesses and the resulting properties of the coatings are compiled in Table 2, under C, resp. A. Additional properties are listed in Table 3. It is found that the properties of the coatings of the formulations C and A, obtained according to this Example 3, are markedly inferior to those of the coatings of formulation F resp. B of Example 2a). Analogous comparison data are obtained for the coatings D, E and G if tris(2-oxo-1,3-dioxolanyl-4-methyl)isocyanurate and ethyltriphenylphosphonium bromide are added separately to the formulation in the comparison test.

**Table 1**

| (Powder coating formulations) | | | | | | | |
|---|---|---|---|---|---|---|---|
| formulation [% by weight] | A | B | C | D | E | F | G |
| | | | | | | | |
| polyester % | CC 801¹⁾ 61.73 | CC 801¹⁾⁾ 61.73 | CC 801¹⁾⁾ 60.66 | CC 801¹⁾⁾ 86.74 | CC 801¹⁾⁾ 60.60 | CC 801¹⁾⁾ 60.67 | CC 801¹⁾⁾ 86.89 |
| PT 910²⁾, %, 3 mm coating thickness | --- | --- | 4.94 | 7.07 | 4.94 | 4.94 | 7.08 |
| TGIC-CO₂ % | 5.63 | --- | 2.21 | --- | --- | --- | --- |
| TGIG-CO₂ with ETPPBr, % | --- | 5.64 5% cat. | --- | 3.49 10% cat. | 2.44 10% cat. | 2.33 5% cat. | 3.33 5% cat. |
| ETPPBr % | 0.28 | --- | 0.12 | --- | --- | --- | --- |
| benzoin % | 1.07 | 1.07 | 1.05 | 1.50 | 1.05 | 1.05 | 1.50 |
| Resiflow PV 88³⁾ % | 0.86 | 0.86 | 0.84 | 1.20 | 0.84 | 0.84 | 1.20 |
| TiO₂ [Kronos 2160]⁴⁾, % | 30.70 | 30.70 | 30.17 | --- | 30.14 | 30.18 | --- |

| | | | | | | | |
|---|---|---|---|---|---|---|---|
| 1) Crylcoat®, of UCB, Belgium; | | | | | | | |
| 2) Ciba Specialty Chemicals, Switzerland, mixture of diglycidyl terephthalate and trimellitic acid triglycidyl ester according to P.-G. Gottis, J.-A. Cotting, FATIPEC Congress (1996), 23^{rd} (Vol.B), B216-B231 (ISSN:0430-222)³⁾; | | | | | | | |
| 3) flow control agent, Worlee | | | | | | | |
| 4) according to Kronos Information of 13.05.94 about Kronos® 2160 TGIC-CO₂ = tris(2-oxo-1,3-dioxolanyl-4-methyl)isocyanurate; ETPPBr = ethyltriphenylphosphonium bromide | | | | | | | |

**Table 2**

| (Properties of the coatings) | | | | | | | |
|---|---|---|---|---|---|---|---|
| | A | B | C | D | E | F | G |
| gel time at 180°C | > 600 s | > 600 s | 110 s | 80 s | 90 s | 240 sec | 140 s |
| cure | 15 min. / 200°C | 15 min. / 200°C | 15 min. / 200°C | 10 min. / 200°C | 10 min. / 200°C | 15 min. / 200°C | 15 min. / 200°C |
| coating thickness [µm] | 57 | 54 | 59 | 61 | 68 | 67 | 64 |
| substrate | Q-panel | Q-panel | Q-panel | Q-panel | Q-panel | Q-panel | Q-panel |
| gloss 60° | 89 | 98 | 88 | 98 | 86 | 96 | 104 |
| Yellowness Index YI | 6.7 | 3.4 | 5.2 | --- | 2.8 | 3.7 | --- |
| flow [note] | 6 | 4 | 12 | 12 | 12 | 10 | 10 |
| rev. impact [kg. cm] | <5 | <5 | 40 | >160 | >140 | >160 | >160 |
| acetone test, 1 min. [note] | 5 | 5 | 3 | 3 | 2 | 3 | 3 |
| aspect | full of bubbles | no bubbles | O.K. | O.K. | O.K. | O.K. | O.K. |
| YI = Yellowing Index | | | | | | | |

**Table 3**

| (Curing at different stoving temperatures) | | | |
|---|---|---|---|
| formulation C: | 15 min./160°C | 15 min./180°C | 15 min./200°C |
| rev. impact [kg.cm] | <5 | 5 | 40 |
| acetone test | 3 | 3 | 3 |
| | | | |
| formulation D: | 10 min./160°C | 10 min./180°C | 10 min./200°C |
| rev. impact [kg.cm] | >160 | >160 | >160 |
| acetone test | 3 | 3 | 3 |
| | | | |
| formulation E: | 10 min./160°C | 10 min./180°C | 10 min./200°C |
| rev. impact [kg.cm] | <5 | 20 | >140 |
| acetone test | 3 | 2 | 2 |
| | | | |
| formulation F: | 15 min./160°C | 15 min./180°C | 15 min./200°C |
| rev. impact [kg.cm] | <5 | 30 | >160 |
| acetone test | 3 | 3 | 3 |
| | | | |
| formulation G: | 15 min./160°C | 15 min./180°C | 15 min./200°C |
| rev. impact [kg.cm] | 15 | >160 | >160 |
| acetone test | 3 | 3 | 3 |

## Claims

1. A crosslinking composition for heat-curable carboxyl-containing polymers, in particular for systems containing carboxyl-terminated polyesters, carboxyl-containing acrylate polymers and/or methacrylate polymers, comprising a crosslinking agent, which consists at least of one cyclocarbonate group-containing isocyanurate compound, and at least one catalyst dissolved or dispersed therein, which has been incorporated separately into the isocyanurate compound prior to the crosslinking reaction.

2. A crosslinking composition according to claim 1, wherein the cyclocarbonate group-containing isocyanurate compound has a melting point of at least 120°C, preferably of at least 130°C and, particularly preferably, of at least 140°C.

3. A crosslinking composition according to claim 1, wherein the cyclocarbonate group-containing isocyanurate compound is a tris(2-oxo-1,3-dioxolanyl-4-methyl)isocyanurate of formula (I):

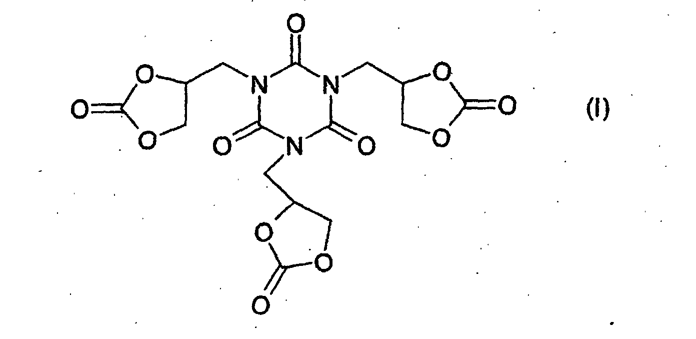

4. A crosslinking composition according to claim 1, wherein the cyclocarbonate group-containing isocyanurate compound is a compound analogous to the compound of formula (I) and contains only one single or two cyclocarbonate group(s), the remaining groups being in the form of glycidyl radicals, which analogous compound preferably contains not more than 35 mol-%, particularly preferably not more than 5 mol-% of epoxy groups.

5. A crosslinking composition according to claim 1, which comprises the catalyst in an amount from 0.01 % by weight to 20 % by weight, preferably from 0.1 % by weight to 10 % by weight, more preferably from 5 % by weight, based on the weight of the cyclocarbonate group-containing isocyanurate compound.

6. A crosslinking composition according to claim 1, which comprises a catalyst which acts selectively predominantly or exclusively on the cyclocarbonate group-containing isocyanurate compound.

7. A crosslinking composition according to claim 1, wherein the catalyst which accelerates the crosslinking reaction of the cyclocarbonate group-containing isocyanurate compounds with the carboxyl-containing polymers is a compound acting as a Lewis acid or as a Lewis base, FeSO₄, NaHSO₄, CeSO_{4,} H₃PO₄, ZnCl₂, Na₂CO₃, phosphonic acid, p-toluenesulfonic acid, dimethylsulfonic acid, an ammonium salt and/or a phosphonium salt.

8. A crosslinking composition according to claim 1, wherein the catalyst which accelerates the crosslinking reaction of the cyclocarbonate group-containing isocyanurate compounds with the carboxyl-containing polymer is a tetraalkyl ammonium halide, an aryl-and alkyl-substituted ammonium halide and/or a phosphonium salt, preferably a phosphonium halide and, most preferably, an ethyltriphenylphosphonium bromide.

9. A process for the preparation of a crosslinking composition according to claim 1, which comprises dissolving a cyclocarbonate group-containing isocyanurate compound in a suitable solvent, dissolving or dispersing therein at least one catalyst and then removing the solvent again.

10. Use of the crosslinking composition according to any one of claims 1-8 as hardener for heat-curable carboxyl-containing polymers, in particular as hardener in systems containing carboxyl-terminated polyesters, carboxyl-containing acrylate and/or methacrylate polymers, preferably in corresponding heat-curable paint systems, in particular powder coating compositions.

## Patentansprüche

1. Vernetzungsmittelzusammensetzung für thermisch härtbare carboxylhaltige Polymere, insbesondere carboxylterminierte Polyester, carboxylhaltige Acrylat- und/oder Methacrylatpolymere, enthaltende Systeme, enthaltend ein Vernetzungsmittel, welches mindestens aus einer Cyclocarbonatgruppen enthaltenden Isocyanuratverbindung besteht, und mindestens einen darin gelösten oder dispergierten Beschleuniger, welcher vor der Vernetzungsreaktion separat in die Isocyanuratverbindung eingearbeitet wurde.

2. Vernetzungsmittelzusammensetzung nach Anspruch 1, **dadurch gekennzeichnet, dass** die Cyclocarbonatgruppen enthaltende Isocyanuratverbindung einen Schmelzpunkt von mindestens 120°C, vorzugsweise mindestens 130°C und vorzugsweise mindestens 140°C aufweist.

3. Vernetzungsmittelzusammensetzung nach Anspruch 1, **dadurch gekennzeichnet, dass** die Cyclocarbonatgruppen enthaltende Isocyanuratverbindung ein tris(2-Oxo-1,3-dioxolanyl-4-methyl)isocyanurat der Formel (I): 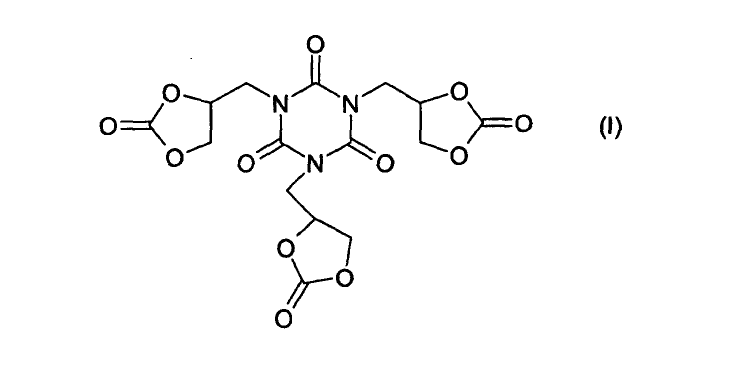 darstellt.

4. Vernetzungsmittelzusammensetzung nach Anspruch 1, **dadurch gekennzeichnet, dass** die Cyclocarbonatgruppen enthaltende Isocyanuratverbindung eine Verbindung analog zur Verbindung der Formel (I) darstellt, und nur eine einzige oder zwei Cyclocarbonatgruppen aufweist und die verbleibenden Gruppen als Glycidylreste vorliegen, wobei diese analoge Verbindung vorzugsweise höchstens 35 Mol-%, und vorzugsweise nicht mehr als 5 Mol-%. an Epoxidgruppen aufweist.

5. Vemetzungsmittelzusammensetzung nach Anspruch 1, **dadurch gekennzeichnet, dass** diese den Beschleuniger in einer Menge von 0.01 Gew.-% bis 20 Gew.-%, vorzugsweise 0.1 Gew.-% bis 10 Gew.-%, und vorzugsweise in einer Konzentration von etwa 5 Gew.-%, bezogen auf das Gewicht der Cyclocarbonatgruppen enthaltenden Isocyanuratverbindung enthält.

6. Vernetzungsmittelzusammensetzung nach Anspruch 1, **dadurch gekennzeichnet, dass** diese einen Beschleuniger enthält, der selektiv vorwiegend oder ausschliesslich auf die Cyclocarbonatgruppen enthaltende Isocyanuratverbindung einwirkt.

7. Vernetzungsmittelzusammensetzung nach Anspruch 1, **dadurch gekennzeichnet, dass** der Beschleuniger, welcher die Vemetzungsreaktion der Cyclocarbonatgruppen enthaltenden Isocyanuratverbindungen mit dem carboxylhaltigen Polymeren beschleunigt, eine als LewisSäure oder als Lewis-Base wirkende Verbindung, FeSO₄, NaHSO₄, CeSO_{4,} H₃PO₄, ZnCl₂, Na₂CO₃, Phosphonsäure, p-Toluol-sulphonsäure, Dimethyl-sulphonsäure, ein Ammoniumsalz und/oder ein Phosphoniumsalz darstellt.

8. Vernetzungsmittelzusammensetzung nach Anspruch 1, **dadurch gekennzeichnet, dass** der Beschleuniger, welcher die Vemetzungsreaktion der Cyclocarbonatgruppen enthaltenden Isocyanuratverbindungen mit dem carboxylhaltigen Polymeren beschleunigt, ein Tetraalkylammoniumhalogenid, ein aryl- und alkylsubstituiertes Ammoniumhalogenid und/oder ein Phosphoniumsalz darstellt, vorzugsweise ein Phosphoniumhalogenid und insbesondere Ethyltriphenylphosphoniumbromid.

9. Verfahren zur Herstellung einer Vemetzungsmittelzusammensetzung nach Anspruch 1, **dadurch gekennzeichnet, dass** man eine Cyclocarbonatgruppen enthaltenden Isocyanuratverbindung in einem geeigneten Lösungsmittel auflöst, darin mindestens einen Beschleuniger löst oder dispergiert und anschliessend das Lösungsmittel wieder entfernt.

10. Verwendung der Vemetzungsmittelzusammensetzung nach einem der Ansprüche 1-8, als Härter für thermisch härtbare carboxylhaltige Polymere, insbesondere als Härter in carboxylterminierte Polyester, carboxylhaltige Acrylat- und/oder Methacrylatpolymere, enthaltenden Systemen, vorzugsweise in entsprechenden thermisch härtbaren Lacken, insbesondere Pulverlacken.

## Revendications

1. Composition de réticulation pour polymères carboxylés thermodurcissables, en particulier pour des systèmes contenant des polyesters à terminaison carboxyle, des polymères acryliques et/ou polymères méthacryliques carboxylés, comprenant un agent de réticulation qui consiste en au moins un isocyanurate contenant au moins un groupe cyclocarbonate et au moins un catalyseur qui y est dissous ou dispersé, qui a été incorporé séparément dans l'isocyanurate avant la réaction de réticulation.

2. Composition de réticulation selon la revendication 1, dans laquelle l'isocyanurate contenant au moins un groupe cyclocarbonate a un point de fusion d'au moins 120°C, de préférence d'au moins 130°C et très préférablement d'au moins 140°C.

3. Composition de réticulation selon la revendication 1, dans laquelle l'isocyanurate contenant au moins un groupe cyclocarbonate est un isocyanurate de tris(2-oxo-1,3-dioxolanyl-4-méthyle) de formule (I) :

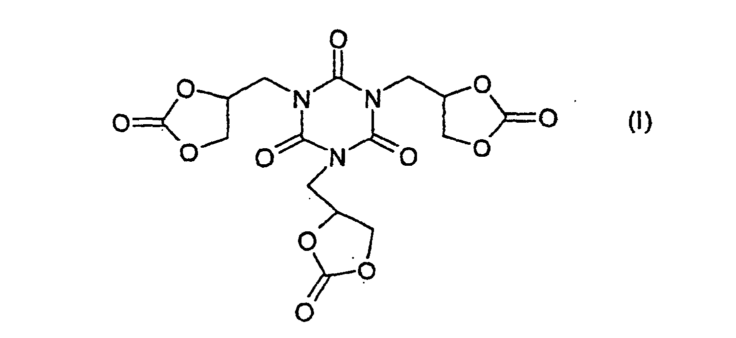

4. Composition de réticulation selon la revendication 1, dans laquelle l'isocyanurate contenant au moins un groupe cyclocarbonate est un composé analogue au composé de formule (I) et ne contient qu'un seul ou deux groupe(s) cyclocarbonate, les groupes restants étant sous la forme de radicaux glycidyle, ce composé analogue ne contenant de préférence pas plus de 35 mol %, très préférablement pas plus de 5 mol %, de groupes époxy.

5. Composition de réticulation selon la revendication 1, qui comprend le catalyseur en une quantité de 0,01 % en poids à 20 % en poids, de préférence de 0,1 % en poids à 10 % en poids, mieux encore de 5 % en poids, par rapport au poids de l'isocyanurate contenant au moins un groupe cyclocarbonate.

6. Composition de réticulation selon la revendication 1, qui comprend un catalyseur qui agit sélectivement, principalement ou exclusivement, sur l'isocyanurate contenant au moins un groupe cyclocarbonate.

7. Composition de réticulation selon la revendication 1, dans laquelle le catalyseur qui accélère la réaction de réticulation des isocyanurates contenant au moins un groupe cyclocarbonate avec les polymères carboxylés est un composé agissant comme un acide de Lewis ou une base de Lewis, FeSO₄, NaHSO₄, CeSO₄, H₃PO₄, ZnCl₂, Na₂CO₃, un acide phosphonique, l'acide p-toluènesulfonique, l'acide diméthylsulfonique, un sel d'ammonium et/ou un sel de phosphonium.

8. Composition de réticulation selon la revendication 1, dans laquelle le catalyseur qui accélère la réaction de réticulation des isocyanurates contenant au moins un groupe cyclocarbonate avec le polymère carboxylé est un halogénure de tétraalkylammonium, un halogénure d'alkyl- et aryl-ammonium et/ou un sel de phosphonium, de préférence un halogénure de phosphonium et très préférablement un bromure d'éthyltriphénylphosphonium.

9. Procédé pour la préparation d'une composition de réticulation selon la revendication 1, qui comprend les étapes consistant à dissoudre un isocyanurate contenant au moins un groupe cyclocarbonate dans un solvant approprié, à y dissoudre ou disperser au moins un catalyseur, puis à éliminer le solvant.

10. Utilisation de la composition de réticulation selon l'une quelconque des revendications 1 à 8 comme durcisseur pour polymères carboxylés thermodurcissables, en particulier comme durcisseur dans des systèmes contenant des polyesters à terminaison carboxyle, des polymères acryliques et/ou méthacryliques carboxylés, de préférence dans des systèmes de peinture thermodurcissables, en particulier des compositions de revêtement en poudre.
